# EUROPEAN PATENT APPLICATION

(11) **EP 4 844 400 A1**
(43) Date of publication of application: **25.11.2026**
(21) Application number: 25382514.5
(22) Date of filing: 22.05.2025
(51) Int. Cl.: G01N 33/03, G01N 21/78, G01N 31/22

(54) **OPTICAL METHOD FOR THE QUANTIFICATION OF THE CONCENTRATION OF THE PHENOLIC COMPOUNDS OLEOCANTHAL AND OLEACEIN**

(71) Applicant: Universidad de Granada, 18071 Granada (ES)
(72) Inventor: MEDINA CASTILLO, Antonio Luis, 18071 Granada (ES); LÓPEZ AVEIGA, Melany Gisell, 18071 Granada (ES); FERNÁNDEZ RAMOS, María Dolores, 18071 Granada (ES); GONZÁLEZ CASADO, Antonio, 18071 Granada (ES); MARTOS NÚÑEZ, Vanesa, 18071 Granada (ES)
(74) Representative: Cueto, Sénida

(57) **Abstract**

The invention refers to an optical method for the direct and selective measurement of the concentration of OC and OLA in a liquid sample which comprises:
- to contact the liquid sample with a calibrated optically sensitive material
- to measure a change in an optical property of the optically sensitive material during a constant reaction time and temperature
- to associate the change measured in the preceding step in the optical property with the total concentration of OC and OLA of the sample using the calibration curve of the optically sensitive material,
particularly for measurement in olive oil.

## Description

### FIELD OF THE INVENTION

The present invention falls within the field of analytical chemistry, more specifically in the area of sensor devices for in situ (at the point of production) quantification of the total concentration of phenolic compounds, particularly the phenolic compounds oleocanthal (OC) and oleacein (OLA), in liquid samples (e.g. any type of vegetable oil, aqueous or organic extracts of plants or fruits).

### BACKGGROUND OF THE INVENTION

In the 1960s, a seven-country study showed that mortality from coronary heart disease in the Mediterranean area was two to three times lower than in northern Europe and the United States. This finding was attributed to the Mediterranean diet whose central feature is the consumption of olive oil as the main source of fat. Traditionally, the types of olive oil consumed have been virgin olive oil (VOO) and extra virgin olive oil (EVOO), which are obtained from the fruit of the olive tree solely by mechanical means, with EVOO having the best physicochemical and organoleptic characteristics. OO and EVOO have a particular fatty acid composition, being rich in monounsaturated oleic acid, and also contain other minor components (around 2% by weight), such as phytosterols, tocopherols, and biophenols. For some time, the high monounsaturated fatty acid content was considered to be responsible for the cardioprotective effects of olive oil. However, it is now known that most of these benefits are also related to the minor components of the unsaponifiable fraction, in particular phenolic compounds (biophenols). Biophenols in olive oil are considered to be responsible for some pharmacological properties such as anti-atherogenic, anti-hepatotoxic, hypoglycaemic, anti-inflammatory, antioxidant, anti-tumour, antiviral, analgesic, purgative, and immunomodulatory activity, as well as protection against neurodegeneration associated with ageing..

In recent years, two of the biophenols of olive oil (oleocanthal: OC and oleacein: OLA) have attracted considerable scientific interest due to their biological properties, in particular their ability to modulate inflammation, oxidative stress, and cell proliferation. Most of this evidence comes from in vitro studies, but some animal studies have also been published. For example, OC has been found to exert anti-inflammatory and antioxidant actions in animal models of lupus and arthritis and OLA has been found to improve adiposity, steatosis, weight gain, and insulin resistance in mice fed with a high-fat diet. Several clinical studies have examined the health benefits of olive oils rich in the biophenols OC and OLA. One of these studies was conducted in 9 healthy individuals and examined the effect of two similar virgin olive oils (one oleocanthal-rich and one oleocanthal-poor) on platelet aggregation. The results of this study suggested acute antiplatelet effects in individuals who had consumed oleocanthal-rich EVOO. In another study, high OC and OLA EVOO was administered to 26 patients with early-stage chronic lymphocytic leukaemia, concluding that high OC and OLA EVOO may be a promising dietary approach for the improvement of this disease. A third study in 23 subjects with metabolic syndrome and hepatic steatosis showed that administration of oleocanthal-rich EVOO for two months had beneficial effects on metabolic parameters, inflammatory cytokines, and abdominal fat distribution. The most recent study, in which the Instituto de la Grasa (IG), a research centre of the Consejo Superior de Investigaciones Cientificas (CSIC) together with the Hospital Regional Universitario de Málaga and the University of Málaga, has participated, shows that Extra Virgin Olive Oil (EVOO) rich in the biophenols OC and OLA can improve the health of people with obesity and in the early stages of diabetes .

All of the above means that the biophenols OC and OLA have become indicators of olive oil quality, and that olive oils from the Mediterranean basin rich in these two biophenols is a product with great therapeutic potential and high added value. Therefore, the quantification of the concentration of OC and OLA is a topic of great interest for the olive oil industry.

There is currently no method capable of selectively quantifying the concentration of OC and OLA in olive oil *in situ* (directly at the point of production: oil mill) in a direct, simple, rapid, cost-effective, and environmentally friendly manner **[1-3].**

Therefore, the determination of the concentration of OC and OLA in olive oil must be carried out by chromatographic techniques in specialised laboratories **[1,2]**. An additional problem is that both OC and OLA are highly reactive molecules that react rapidly with the water or methanol used in the mobile phase, resulting in broadened or multiple chromatographic peaks **[3].** Because of this, the determination of OC and OLA by chromatographic techniques requires purification processes involving long and complex derivatization reactions, resulting in long analysis times and excessively high prices **[3].** All this means that, at present, due to the difficulties involved in chromatographic methods of analysis of OC and OLA in olive oil, enhancing the therapeutic qualities of olive oils from the Mediterranean basin through the added value provided by these biophenols, as well as exhaustively studying the variables that affect their concentration (climate, type of soil, olive processing, etc.), is a very difficult and extremely costly task.

The present invention solves the problems of the quantification of the biophenols OC and OLA in liquid samples (e.g. any type of vegetable oil, aqueous or organic extracts of plants or fruits), by developing an optical sensor that allows their selective and *in-situ* detection (at the point of production), in a simple, direct, environmentally friendly, and inexpensive way.

According to the present invention, an optical method is provided that allows the selective and *in situ* quantification of the concentration of OC and OLA in liquid samples (e.g. any type of vegetable oil, aqueous or organic extracts of plants or fruits), in a direct, fast, environmentally friendly, and cost-effective way.

Due to the extraordinary therapeutic properties of the biophenols OC and OLA, the olive oil rich in these biophenols are considered foods with extraordinary therapeutic potential. Due to this, the quantification of the concentration of OC and OLA in olive oils has become a priority for the olive oil industry in the Mediterranean basin. However, currently the quantification of OL and OLA is carried out using chromatographic methods, that have a series of disadvantages, such as: they do not allow measurements to be made *in situ,* so they require the collection, transport, and storage of samples, expensive equipment, specialized technicians, tedious and lengthy sample purification processes are required, they are not environmentally friendly as they require a high consumption of toxic organic solvents, etc.

To the best of our knowledge, the method of this invention is the first optical method capable of selectively and accurately *in situ* quantifying the total concentration of OC and OLA in a simple (by incubating the optically sensitive material directly in the oil sample), cost-effective (ilt does not require expensive equipment and specialized techniques), and environmentally friendly (not require additional reagents and large amount of organic solvents) way.

### DESCRIPTION OF THE INVENTION

The phenolic compounds oleocanthal (OC) and oleacein (OLA) are two key secoiridoids derivatives present in virgin olive oil (VOO) and extra virgin olive oil (EVOO) that have become of great clinical and nutritional interest due to their demonstrated bioactivity.

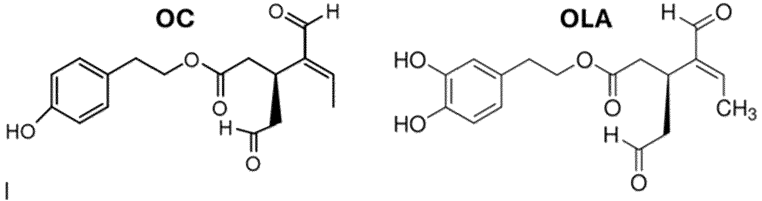

The inventors of the present invention have found that in olive oil there are only two phenolic compounds (oleocanthal: OC and oleacein: OLA) with reactive aldehyde groups. The reactive aldehyde groups of OC and OLA can react with other functional groups to form molecules with optical properties, e.g. with amino groups to form imines (Schiff bases) and enamines with optical properties (color, absorbance, fluorescence, etc).

This invention exploits the reactivity of aldehyde groups of OC and OLA to the formation of molecules with optical properties to develop an optical method for the *in situ* selective quantification of OC and OLA in liquid samples, preferably vegetable oils and aqueous or organic extracts of plants or fruits.

In this invention, the formation of molecules with optical properties is carried out in heterogeneous phase by a reaction between the aldehyde groups of OC and OLA and the reactive functional groups (e.g. amine groups) comprised in an optically sensitive material.

For the development of the method of the invention, it is necessary to establish the relationship between the total concentration of OC and OLA in the sample and the change in the optical property of the optically sensitive material when the reaction between the aldehyde groups of OC and OLA and the functional groups of the optically sensitive material is produced. Based on these principles, the formation of molecules with optical properties on the surface of optically sensitive material can be considered as heterogeneous phase reaction.

So, this invention provides an optically sensitive material that allows to establish a relationship (calibration curve) between the total concentration of OC and OLA in the sample and the intensity of an optical property of the optically sensitive material.

The object of the invention is an optical method for the direct and selective measurement of the concentration of OC and OLA in liquid samples which comprises:
- to contact a liquid sample with a calibrated optically sensitive material
- to measure a change in an optical property of the optically sensitive material during a constant reaction time and temperature
- to associate the change measured in the preceding step in the optical property with the total concentration of OC and OLA of the sample using the calibration curve of the optically sensitive material.

The term "calibrated" has its usual meaning, i.e., the readings or results that are obtained from the optically sensitive material have been correlated with those of a standard. The calibration process is explained below.

According to particular embodiments, the method comprises to carry out a sample pre-treatment to remove carboxylic compounds present in the liquid sample, before contacting the sample with the calibrated optically sensitive material. The removal of those groups can be carried out, for example, by making them to react with a strong base, such as tertiary amines.

According to particular embodiments, when the sample is olive oil, the method comprises to carry out a pre-treatment to remove carboxylic compounds, that comprises:
- to prepare polymeric microparticles functionalized with tertiary amine groups,
- to make the olive oil to react with the polymeric microparticles functionalized with tertiary amine groups
- to filtrate the obtained polymeric microparticles upon reaction with the olive oil sample, thus removing the portion of the sample that interferes with the measurement of the concentration of OLA and OC.

The polymeric microparticles can be prepared, for example following the protocol disclosed in **[6, 7].**

The optically sensitive material comprises at least one reactive functional group able to react with aldehyde groups of OC and OLA to form molecules with optical properties.

In a particular embodiment, such reactive functional groups are amine, thiol, carboxy or hydroxyl.

The time of reaction is between 15 and 60 min, preferably between 20 and 60 min. The temperature of reaction is comprised between 20 and 80°C, preferably, between 20 and 60°C.

In a preferred operation mode, the procedure of the invention maintains the reaction at a temperature comprised between 20 and 60°C during a time comprised between 15 and 50 min.

### Optically sensitive materials.

The optically sensitive material can be a reagent containing functional groups capable of reacting with the aldehyde groups of OC and OLA.

The optically sensitive material selected from:
- organic, inorganic or hybrid organic/inorganic materials that intrinsically possess functional groups capable of reacting with the aldehyde groups of OC and OLA to form chemical groups with optical properties
- organic, inorganic or hybrid organic/inorganic materials that do not naturally have functional groups capable of reacting with the aldehyde groups of OC and OLA to form chemical groups with optical properties, functionalized with reactive functional groups capable of react with the aldehyde groups of OC and OLA to from chemical groups with optical properties. In this case, the organic, inorganic or hybrid organic/inorganic material can be considered as a solid support for the functional groups capable of reacting with the aldehyde groups.

In a preferred embodiment, the solid support is selected among porous or non-porous organic, inorganic or hybrid (organic-inorganic), based materials.

According to particular embodiments, the optically sensitive materials of the invention can be selected from organic, inorganic or hybrid (organic/inorganic) materials that possess functional groups, naturally of that are functionalized with them, capable of react with the aldehyde groups of OC and OLA to form molecules with optical properties like coloured imines (Schiff bases) or enamines by reaction between the aldehyde groups of OC and OLA and amine groups of the optically sensitive materials.

The optically sensitive material can be zero-dimensional (0D: 0D means the group of materials that have all three dimensions less than 100 nm), one-dimensional (1D: 1D means the group of materials that have two dimensions less than 100 nm and one dimension higher than 100 nm), two-dimensional (2D: 2D means the group of materials that have one dimension less than 100 nm and two dimensions higher than 100 nm) or three-dimensional (3D: means the group of materials that have all three dimensions higher than 100 nm). Therefore, optically sensitive material can be shaped as membranes, nanoparticles, microparticles, fibers, and others.

Examples of optically sensitive materials, are: 0D, 1D, 2D or 3D materials made of , or comprising, different types of polymers such a polysaccharides (cellulose, starch, alginate, chitin, chitosan, pectin, agar-agar, hydroxyethyl cellulose, carboxymethylcellulose, methylcellulose, nitrocellulose, and others), Nylon, polyimides, methacrylates, acrylates, polyacrylamides, polystyrenes, polymethacrylates, polyacrylates, silicones, polyamides, polyesters, polycarbonates, polyethylenes, polyacetylenes, polypropylenes, polyacrilonitriles, tetrafluoroethylenes, polytetrafluoroethylenes, polyurethanes, polysulfones, polythiophenes, polyanilines, polypyrroles, polybenzimidazoles, polyketones, polyamines, DNA, proteins, colloids (carbon dots, quantum dots, metal colloids, and others), when appropriate, functionalized with reactive groups -preferably, amine groups -, hybrid materials such as mixtures of the previous ones, when appropriate functionalized with reactive groups, preferably, amine groups.

Additional examples of optically sensitive materials, are: 0D, 1D, 2D o 3D materials made of polymers that intrinsically (without the need for any functionalization) possess amino groups such as Poly(allylamine), polyethylenimine, polyurethanes, chitosan, and proteins.

According to a preferred embodiment, the solid support is a cellulose membrane, which is functionalized with amine molecules capable of forming imines (Schiff bases) or enamines with optical properties.

The optically sensitive material of the invention needs to be able to respond by means of a change in any of its optical properties to small changes in the concentration of OC and OLA.

The optical property of the optically sensitive material can be selected among color, transmittance, absorbance, reflectance, luminescence (with its many modes: intensity or polarization, decay time, energy transfer, and quenching efficiency), infrared and Raman spectroscopies, interferometry, and surface plasmon resonance.

A reaction in heterogenous phase takes place according to the method of the invention, in optically sensitive materials. Optically sensitive materials are made up of a component or components that have functional groups capable of reacting with aldehyde groups of OC and OLA to form molecules with optical properties. As an example, a natural polymer such as cellulose, functionalized with primary or secondary amine groups capable of reacts with aldehyde groups of OC and OLA to form colored imines (Schiff bases) or enamines. Therefore, optically sensitive materials can be a membrane or any other material (0D, 1D, 2D o 3D), directly manufactured with cellulose, and functionalized with amine groups.

*"Reaction in heterogenous phase"* means reactions in which the reactants are components of two or more phases (solid and gas, solid and liquid, two immiscible liquids) or in which one or more reactants undergo chemical change at an interface, e.g., on the surface of a solid catalyst.

According to particular embodiments, amine molecules for the manufacture of optically sensitive materials are selected from primary and secondary, preferably: ethylenediamine, propylenediamine, poliethylenimine, poly(ethylene glycol) bis(amine), hexamethylenediamine, p-phenylenediamine, m-xylylenediamine, tris(2-aminoethyl)amine, diethylenetriamine, triethylenetetramine, 3,3'-diamino-N-methyldipropylamine, poly(allylamine), spermidine, cadaverine, spermine, thermospermine, caldopentamine, caldohexamine, branched and hyperbranched polyamines, aromatic amines, polycyclic aromatic amines, hydroxyl amines, thiol amines, carboxyl amines.

In the case of optically sensitive materials functionalised with amine molecules, the functionalisation can take place through covalent interactions, electrostatic interactions, trapping, hydrophobic interactions and encapsulation.

The optically sensitive inorganic-based materials can be, or can comprise, one or more of the following raw materials, in all cases functionalised with a compound that possess functional groups capable of reacting with the aldehyde group, such as: glass, aluminium oxide, silicon dioxide, zirconium oxide, zeolites, carbon, cordierite, silicon carbide, silicon nitride, mullite, bentonite, zeolite, aluminium oxide, tin oxide, silicon carbide, iron oxide, zinc oxide, silicon, zirconium, copper, gold, silver, titanium, and iron.

Optically sensitive hybrid-based materials include those made from a mixture of organic and inorganic materials, in all cases functionalised with a compound that possess functional groups capable of reacting with the aldehyde group, such as Metal Organic Framework (MOF), composite materials based on inorganic micro or nanoparticles dispersed in organic polymers or vice versa.

The manufacture of optically sensitive materials can be carried out by using one or more of the materials mentioned in the preceding paragraphs.

### Calibration of the optically sensitive materials

The calibration of the optically sensitive material is carried out by measuring the change of an optical property of the optically sensitive material against the concentration of OC and OLA contained in standard solutions prepared in an OC- and OLA-free oily matrix to obtain a calibration curve.

The calibration of the optically sensitive material is performed in a liquid matrix free of OC and OLA, said matrix can be selected among refined olive oil, sunflower oil, triolein, water, organic solvents: methanol, ethanol, acetonitrile, hexane, mixtures of organic solvents, and mixtures of organic solvents and water.

The sample is any type of liquid (e.g. any type of vegetable oil, and aqueous or organic extracts of plants or fruits).

### Optical Properties measurement

The optical property can be measured with any commercial instrument (such as colorimeter, photometer, spectrometer, fluorimeter, reflectometer).

The total concentration of OC and OLA is measured in the same way: by contacting the optically sensitive material with the sample and subsequently measuring the change in the optical property produced due to the reaction of the aldehyde groups of OC and OLA and the functional groups of the optically sensitive material.

For each type of liquid sample, the matrix used for calibration of the optically sensitive material has to be as similar as possible to the liquid sample to be analysed.

For example, the total concentration of OC and OLA can be measured according to a particular embodiment, by introducing a membrane - as an optically sensitive material with functional amine groups - directly in an olive oil sample or into an olive oil sample diluted with organic solvent (preferably THF), incubate the membrane in the sample for 50 min at 60°C to produce the reaction between aldehyde groups of OC and OLA and amine groups of the membrane to form colored imines (Schiff bases) or enamines, wash the membrane with hexane to remove oil residues, and then its color change is measured using a commercial device (colorimeter, photometer, fluorimeter, reflectometer), and the result is extrapolated by means of a previously prepared calibration curve with OC and OLA in refined olive oil (olive oil free of OC and OLA).

### Sample Pre-treatment

In other particular embodiment, the procedure of the invention comprises also a previous step consisting in the pre-treatment of the sample.

The sample may be subjected to a pre-treatment to remove any compound that can interfere in the reaction between the aldehyde groups of OC and OLA and the functional groups of the optically sensitive material.

This step is mandatory when the samples are olive oil samples. So, in a preferred embodiment, where the liquid sample is a sample of olive oil, the process also comprises this step.

### Device of the Invention

In a second aspect, the invention discloses a device for the direct and selective measurement of the concentration of OC and OLA in liquid samples which comprises:
- an optically sensitive material suitably calibrated for the optical measurement of OC and OLA.
- means to bring the optically sensitive material into contact with the liquid samples,
- a thermostatic device with stirring to carry out the reaction between the functional groups of the optically active material capable of reacting with an aldehyde group, and the aldehyde groups of OC and OLA at a controlled temperature between 20°C and 80°C, for a time between 15 and 50 min.
- an optical measurement device capable of measuring the change in the optical property of the optically sensitive material after interaction with the sample
- means to execute a computer program, or processing means to compute the change in the optical property, capable of directly relating the change in the optical property of the optically sensitive material to the concentration of OC and OLA in the sample.

According to particular embodiments, the device comprises:
- an optically sensitive material that comprises amine groups able to react with aldehyde groups of OC and OLA to form molecules with optical properties,
- glass vials (for example, of between 3 and 4 ml) to bring the optically sensitive material into contact with the liquid samples.
- a thermostatic device with stirring capability to carry out the reaction between the amino groups of the optically active material and the aldehyde groups of OC and OLA at a controlled temperature between 20°C and 80°C, for a time between 15 and 50 min.
- an optical measurement device capable of measuring the change in the optical property of the optically sensitive material after interaction with the sample.

### References:

**[1]** Leonardo Di Donna, Hicham Benabdelkamel, Fabio Mazzotti, Anna Napoli, Monica Nardi, Giovanni Sindona. High-Throughput Assay of Oleopentanedialdheydes in Extra Virgin Olive Oil by the UHPLC-ESI-MS/MS and Isotope Dilution Methods. Anal. Chem. 2011, 83, 1990-1995, https://doi.org/10.1021/ac200152r.

**[2]** Verónica Sánchez de Medina, Hristofor Mihod, Eleni Melliou, Prokopios Magiatis, Feliciano Priego-Capote, Maria Dolores Luque de Castro. Quantitative method for determination of oleocanthal and oleacein in virgin olive oils by liquid chromatography-tandem mass spectrometry. Talanta 162 (2017) 24-31, http://dx.doi.ora/10.1016/i.talanta.2016.09.056.

**[3]** Evangelia Karkoula, Angeliki Skantzari, Eleni Melliou, Prokopios Magiatis. Direct Measurement of Oleocanthal and Oleacein Levels in Olive Oil by Quantitative 1H-NMR. Establishment of a New Index for the Characterization of Extra Virgin Olive Oils. J. Agric. Food Chem. 2012, 60, 11696-11703, https://doi.org/10.1021/jf3032765.

**[4]** A.L. Medina-Castillo, L. Ruzic, B. Nidetzky, Juan M. Bolivar. Hydrophilic nonwoven nanofiber membranes as nanostructured supports for enzyme immobilization, ACS Appl. Polym. Mater. 4 (2022) 6054-6066, https://doi.org/10.1021/acsapm.2c00863.

**[5]** Melany G. López Aveiga, Maria Dolores Fernández Ramos, Vanesa Martos Núnez, Luis Fermin Capitán Vallvey, Antonio González Casado, Antonio L. Medina Castillo. Portable fiber-optic sensor for simple, fast, cost-effective, and environmentally friendly quantification of total acidity in real-world applications, Sensors and Actuators: B. Chemical 417 (2024) 136214, https://doi.org/10.1016/j.snb.2024.136214.

**[6]** Antonio L. Medina-Castillo. Thermodynamic Principles of Precipitation Polymerization and Role of Fractal Nanostructures in the Particle Size Control, Macromolecules 2020, 53, 14, 5687-5700, https://doi.org/10.1021/acs.macromol.0c00973.

**[7]** Antonio L. Medina-Castillo, Günter Mistlberger, Jorge F. Fernandez-Sanchez, Antonio Segura-Carretero, Ingo Klimant, Alberto Fernandez-Gutierrez. Novel Strategy To Design Magnetic, Molecular Imprinted Polymers with Well-Controlled Structure for the Application in Optical Sensors, Macromolecules 2010, 43, 1, 55-61, https://doi.ora/10.1021/ma902095s.

### BRIEF DESCRIPTION OF DRAWINGS

**Fig.1****.** Activation of the hydroxyl groups of a paper with vinyl sulfone groups **(A),** preparation of Op-Sens-Mat-ED by covalent immobilization of ED in the cellulose membrane activated with vinyl sulfone groups **(B),** preparation of Op-Sens-Mat-PEI by covalent immobilization of PEI in the cellulose membrane activated with vinyl sulfone groups **(C).**
**Fig.2****.** Optical response (color change from white to red) to oleocanthal concentration of Op-Sens-Mat-ED (A), and Op-Sens-Mat-PEI (B).
**Fig.3****.** Calibration protocol scheme.
**Fig.4****.** Color coordinates of Op-Sens-Mat-PEI in the RGB and SVH spaces versus the concentration of standard solution of OC.
**Fig.5****.** Logarithmic calibration curve **(A),** and its linear representation: (H₀-H) vs Ln [OC] **(B).**
**Fig.6****.** Calibration curves 1 (□: 100% OLA), 2 (○: OC/OLA 50/50 w/w), 3 (X: OC/OLA 75/25 w/w), and 4 (Δ:100% OC).
**Fig.7****.** Scanning electron microscopy (SEM) image of Part-DVB-N-(CH₃)₂.
**Fig.8****.** Blocking of Op-Sens-Mat-PEI by acid-base interaction between the carboxylic compounds (R-COOH) of olive oil and the amino groups of Op-Sens-Mat-PEI **(A),** Pre-treatment of olive oil samples with Part-DVB-N-(CH₃)₂ to remove carboxylic compounds **(B).**
**Fig.9****.** Complete schematic of the EVOO sample measurement protocol.

### EXAMPLE. QUANTIFICATION OF THE CONCENTRATION OF OC AND OLA IN EXTRA VIRGIN OLIVE OIL (EVOO) SAMPLES

In this example, two optically sensitive materials for the quantification of the concentration of OC and OLA in olive oil samples are developed. The optically sensitive materials are based on the functionalization of a porous cellulose paper membrane with amino groups. To introduce the amino groups, the cellulose paper was functionalized with ethylenediamine (Op-Sens-Mat-ED) and Poly(ethyleneimine) (Op-Sens-Mat-PEI). The optical recognition mechanism of Op-Sens-Mat-ED and Op-Sens-Mat-PEI is based on the reaction between the aldehyde groups of OC and OLA and the amino groups of Op-Sens-Mat-ED and Op-Sens-Mat-PEI to forming colored imines (Schiff bases) or enamines. Based on these principles, the formation of imines (Schiff bases) and enamines on the surface of Op-Sens-Mat-ED and Op-Sens-Mat-PEI can be considered as an acid-catalyzed heterogeneous phase reaction that depends on the following experimental variables: **i)** Concentration of OC and OLA in the sample, **ii)** Catalytic concentration of acid, **iii)** Reaction time, **iv)** Physicochemical properties of the optically sensitive material (type of material, polarity, porosity, concentration and type of amine groups, thickness, etc.), and **v)** Temperature. Therefore, by fixing the variables **ii), iii), iv),** and **v),** it is possible to establish a useful relationship between the total concentration of OC and OLA in the sample and the change of the color of the optically sensitive materials due to the formation of colored imines (Schiff bases) or enamines.

### 1. Chemical and Materials.

Divinyl sulfone (DVS), ethylene diamine (ED), poly(ethyleneimine) (PEI), oleocanthal (OC), oleacein (OLA), divinylbenzene (DVB), 2-(Dimethylamino) ethyl methacrylate (DAEM), 2,2'-Azobis(2-methylpropionitrile) (AIBN), acetonitrile (ACN), tetrahydrofuran (THF), methanol (MeOH), dimethyl sulfoxide (DMSO), 2-propanol (2-PrOH), glacial acetic acid (AA), sodium carbonate (SC), potassium dihydrogen phosphate (PDP), were purchased from Sigma-Aldrich, cellulose filter paper (ref. 13058: 73 gm⁻² and 170 µm thickness) from FILTER·LAB^{®} (Barcelona, Spain). Throughout the experiment, all aqueous solutions were prepared using reverse osmosis-type quality water (Milli-RO 12 plus Milli-Q station from Millipore, conductivity 18.2 µΩ·cm).

### 2. Fabrication of two optically sensitive materials

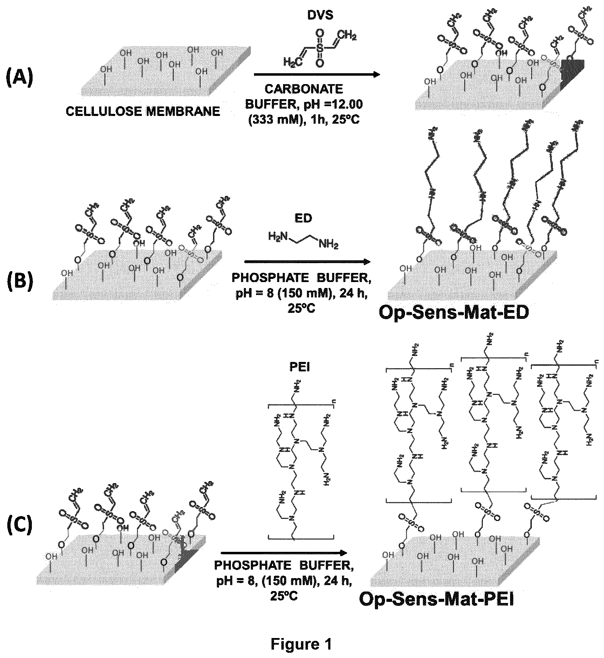

Two examples of the fabrication of optically sensitive materials were developed. The optically sensitive materials were fabricated by the functionalization of a cellulose porous paper membrane with ethylenediamine: ED (Op-Sens-Mat-ED), and Poly(ethyleneimine): PEI (Op-Sens-Mat-PEI). The functionalization was carried out in two steps **[4, 5]** (see **Fig.1**): ***i)** Activation of the hydroxyl groups of the cellulose paper with vinyl sulfone groups.* For this, a piece of cellulose paper (8 cm × 2.3 cm) was introduced into 40 mL of a solution of divinyl sulfone (0.333 M) in sodium carbonate buffer (333 mM) at pH=12.00 for 1h, ***ii)** Covalent immobilization of ED and PEI in the cellulose paper activated with vinyl sulfone groups.* The covalent immobilization of ED and PEI was done by Michael reaction between the vinyl sulfone groups of the activated cellulose membrane and the amine groups of ED and PEI. For this purpose, two pieces (8 cm × 2.3 cm) of cellulose paper activated with vinyl sulfone groups were prepared according to step i). Subsequently, 40 ml solution of each amine (ED and PEI) was prepared at a concentration of 0.25 M in phosphate buffer (150 mM) at pH=8. Finally, the pieces of cellulose paper activated with vinyl sulfone were incubated in the ED and PEI solutions for 24 hours.

### 3. Study of the optical response of Op-Sens-Mat-ED and Op-Sens-Mat-PEI to the concentration of OC

The optical response of Op-Sens-Mat-ED and Op-Sens-Mat-PEI was studied by incubating them for 3 h at room temperature in a standard solution of OC prepared in olive oil free of OC and OLA (refined olive oil). For this purpose, Op-Sens-Mat-ED and Op-Sens-Mat-PEI were cut into circles of 7 mm diameter. On the other hand, a standard solution of 110 mg Kg⁻¹ of OC in refined olive oil was prepared according to the following protocol: First, a stock solution of 1000 mg/l⁻¹ oleocanthal in DMSO was prepared by dissolving 5 mg oleocanthal in 5 ml dimethyl sulfoxide (DMSO). Then, 26.3 µl of the 1000 mg/l⁻¹ OC stock solution was added to a 4 ml glass bottle containing 0.25 ml of refined olive oil. To initiate the imines (Schiff bases) formation, a piece of 7 mm diameter of each material (Op-Sens-Mat-ED and Op-Sens-Mat-PEI) was placed in 0.25 ml of the standard solution of 110 mg Kg⁻¹ of OC, subsequently, 1.5 ml of tetrahydrofuran (THF) and 17.5 µl of acetic acid were added, and the membranes were incubated under shaking at 25°C for 3 h. After incubation, the membranes were washed with 1 ml hexane for 20 sg to remove oil residues.

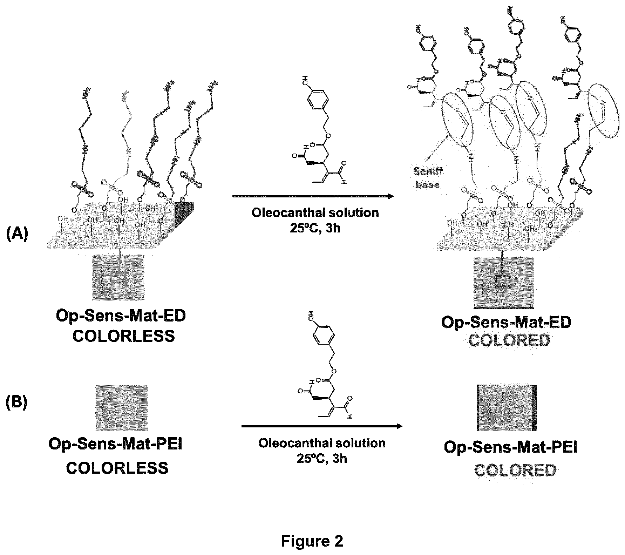

As shown in **Fig. 2****,** the formation of Schiff bases between the amino groups of Op-Sens-Mat-ED and Op-Sens-Mat-PEI and the aldehyde groups of OC causes a change in the color (from white to red) of the membranes, with Op-Sens-Mat-PEI showing a higher response (color intensity), which may be due to its higher concentration of amino groups.

### 4. Experimental optimization of the optical response of Op-Sens-Mat-PEI against the concentration of OC

The imines (Schiff bases) formation between the amino groups of Op-Sens-Mat-ED and Op-Sens-Mat-PEI and the aldehyde groups of the OC and the OLA is an acid-catalyzed reaction. Therefore, considering that olive oil is hydrophobic and immiscible with water, the most important variables that can affect the imines (Schiff bases) formation are: **i)** Temperature, **ii)** Reaction time, **iii)** Organic solvents (Dilution of the oil with organic solvents enhances the formation reaction of Schiff bases since it decreases the viscosity of the oil and increases its polarity, which improves the solubility and diffusion of the acid catalyst, **iv)** Acid concentration.

To optimize the variables that affect the formation of Schiff bases in solid phase mentioned above, Op-Sens-Mat-PEI was selected since this material presents a greater optical response (color intensity) than Op-Sens-Mat-ED (see **Fig. 2**).

The optical response of Op-Sens-Mat-PEI against the variables mentioned above was studied in three OC standard solutions of different concentrations (38 mgKg⁻¹, 110 mgKg⁻¹, 369 mgKg⁻¹). The standard solutions of OC were prepared in refined olive oil using the following protocol: First, a 1000 mg/l⁻¹ oleocanthal stock solution in DMSO is prepared by dissolving 5 mg of oleocanthal in 5 ml of dimethyl sulfoxide (DMSO). To prepare the three standard solutions (38 mgKg⁻¹, 110 mgKg⁻¹, 369 mgKg⁻¹) of oleocanthal in refined olive oil, 8.75 µl, 26.3 µl, and 87.5 µl of the 1000 mg/l⁻¹ oleocanthal stock solution are added to three 4 ml glass bottles, and then 0.25 ml of refined olive oil is added to each vial and the solutions are homogenized for a few seconds by vortexing.

### 4.1. Study of the experimental variables that affect the formation of Schiff bases in solid phase:

*i) Temperature.* Three different temperatures (25°C, 40°C and 60°C) were studied. For this, Op-Sens-Mat-PEI was cut in circular pieces of 7 mm of diameter (membranes). Then, the membranes were introduced in triplicate into the three standard OC solutions, and subsequently, 1.5 ml of tetrahydrofuran (THF) and 17.5 µl of acetic acid were added. Finally, the membranes were incubated under shaking in each of the standard OC solutions at 25°C, 40°C, and 60°C for 3 h. After incubation, the membranes were washed with 1 ml hexane for 20 sg to remove oil residues. The largest color change was observed at 60°C (see **Table 1**).
*ii) Reaction time.* Three different reaction times (20, min, 40 min, and 50 min) were studied. For this purpose, Op-Sens-Mat-PEI was cut in circular pieces of 7 mm of diameter (membranes). The membranes were then introduced in triplicate into the three standard OC solutions, and subsequently, 1.5 ml of tetrahydrofuran (THF) and 17.5 µl of acetic acid were added. Finally, the membranes were incubated under shaking at 60°C, for 30 min, 40 min, and 60 min. After incubation, the membranes were washed with 1 ml hexane for 20 sg to remove oil residues. The largest color change was observed at 60 min (see **Table 1**).
*iii) Organic solvent.* Three different organic solvents: tetrahydrofuran (THF), acetone (ACT), and dimethyl sulfoxide (DMSO) were studied. For this, Op-Sens-Mat-PEI was cut in circular pieces of 7 mm diameter (membranes). The membranes were then introduced in triplicate into the three standard OC solutions, and subsequently, 1.5 ml of the corresponding organic solvent and 17.5 µl of acetic acid were added. Finally, the membranes were incubated under shaking for 60 min, at 60°C. After incubation, the membranes were washed with 1 ml hexane for 20 sg to remove oil residues. The largest colour change was observed in THF (see **Table 1**).
*iv) Acid concentration.* Three concentrations of acetic acid (1% v/v, 2.5% v/v, and 5% v/v) were studied. Firstly, Op-Sens-Mat-PEI was cut into circular pieces of 7 mm diameter (membranes). The membranes were then introduced in triplicate into the three standard OC solutions and subsequently 1.5 ml of THF and the corresponding amount of acetic acid were added. Finally, the membranes were incubated under shaking for 60 min at 60 °C. After incubation, the membranes were washed with 1 ml of hexane for 20 s to remove oil residues. The acid concentration did not affect the color change (see **Table 1**).

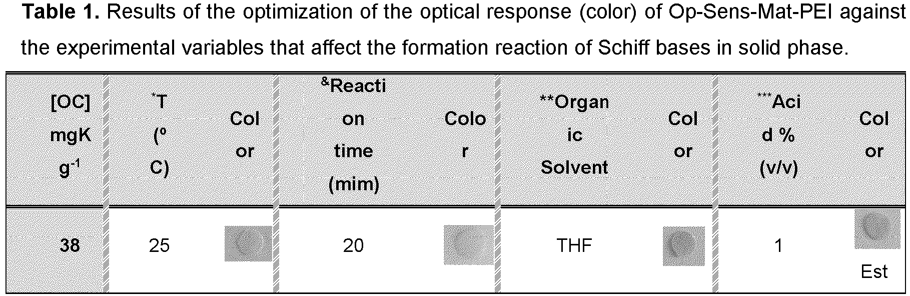

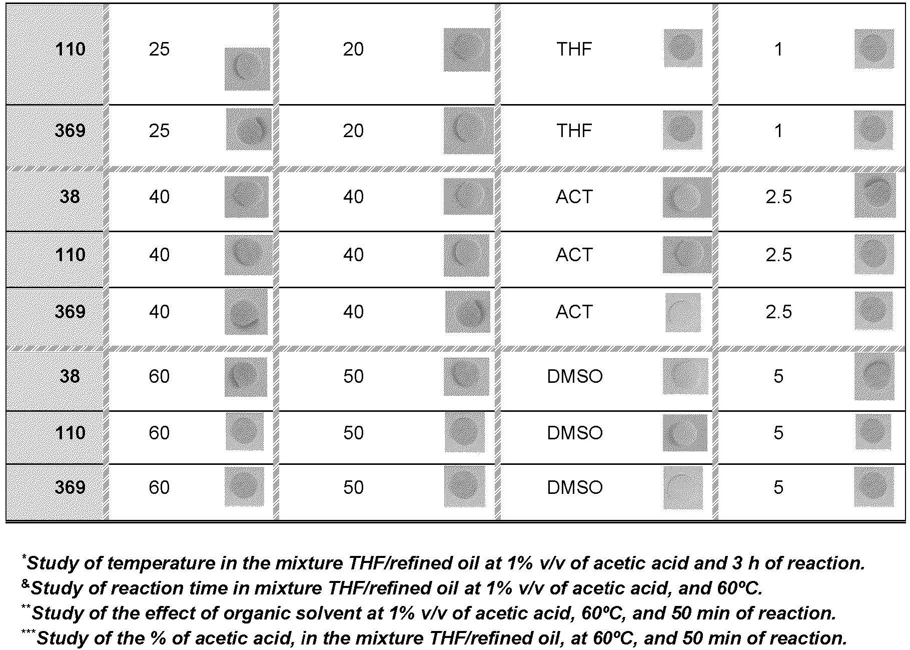

### 5. Calibration of the optical response of Op-Sens-Mat-PEI against the concentration of OC and OLA and mixtures OC/OLA

*5.1. Calibration experimental conditions.* Based on the results of **Table 1,** the optimum experimental condition selected for the formation of colored imines (Schiff bases) on Op-Sens-Mat-PEI by reaction between the aldehyde groups of OC and OLA and the amine groups of Op-Sens-Mat-PEI are: Temperature = 60°C, reaction time = 50 min, 1 % acetic acid v/v, organic solvent = THF.

*5.2. Preparation of standard solutions of OC*. For the OC calibration, 6 standard solutions of OC are prepared in the mixture THF/refined oil, 7/1 v/v with the following final concentrations of OC: 0 mgl⁻¹ (control), 5 mgl⁻¹, 15 mgl⁻¹, 30 mgl⁻¹, 50 mgl⁻¹, and 60 mgl⁻¹. The standard solutions of OC were prepared using the following protocol: First, a 1000 mg/l⁻¹ oleocanthal stock solution in DMSO is prepared by dissolving 5 mg of oleocanthal in 5 ml of dimethyl sulfoxide (DMSO). Then, 0 µl, 8.75 µl, 26.3 µl, 52.5 µl, 87.5 µl, and 105. 5 µl of the 1000 mg/l⁻¹ oleocanthal stock solution are added to five 4 ml glass bottles, and then 0.25 ml of refined olive oil, 1.5 ml of THF and 17.5 µl of acetic acid are added to each vial and the solutions are homogenized for a few seconds by vortexing. Subsequently, a circular piece of 7 mm diameter of Op-Sens-Mat-PEI is introduced in each standard solution of OC and incubated under shaking for 50 min at 60°C. Finally, the membranes were washed with 1 ml of hexane for 20 s to remove oil residues.

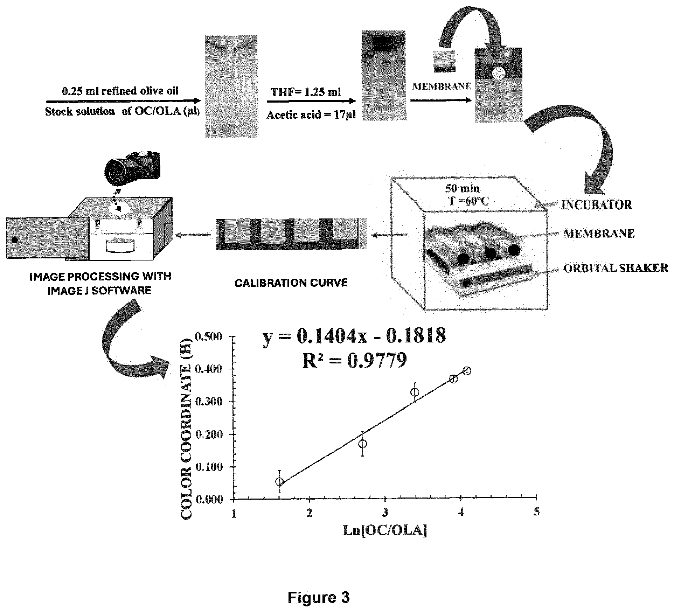

*5.3. Setup for colorimetric measurements: establishing of the calibration curve.* The colorimetric calibration of the optical response of Op-Sens-Mat-PEI against the concentration of OC and OLA is performed using a Canon Powershot G12 digital camera (Japan) placed inside of a homemade wooden enclosure illuminated with two LED lamps (4.6 W, 6000 K, illumination inside of the box = 9680 Lx) positioned at 45° to the digital camera to minimize any interference from external light. The optimized settings used to photograph the membrane were ISO 80, 5.6, shutter speed 1/1600 s, aperture value f/8, focal distance 11 mm; white balance, automatic; resolution, 3648×2432; mode, macro. To evaluate the color change, a photograph was taken in JPEG format. Subsequently, RGB and HSV color space coordinates were obtained from the region of interest (ROI) of the digitized membrane using Image J software. **Fig. 3** shows a schematic of an example of the calibration protocol.

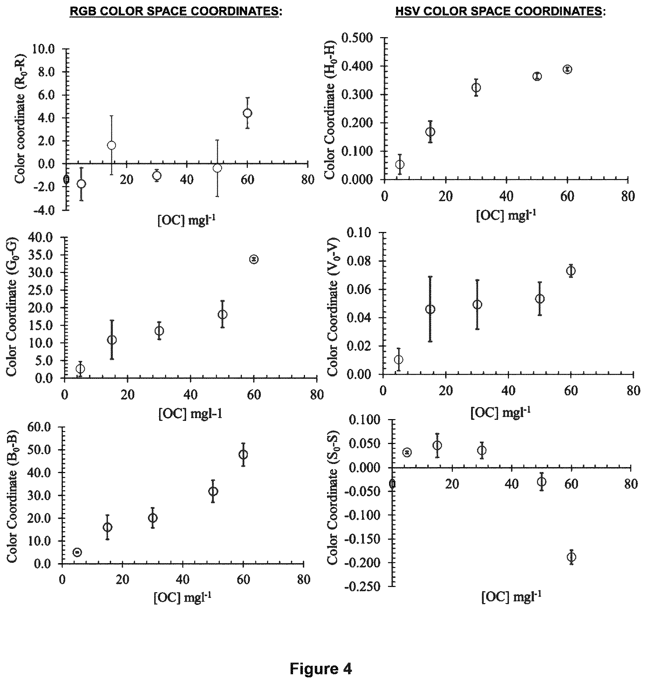

**Fig.4** shows the color coordinates of Op-Sens-Mat-PEI in the RGB and SVH spaces versus the concentration of standard solutions of OC. The analytical signal was obtained by subtracting the value of the chromatic coordinate of the control (0 mgl⁻¹ OC) from each of the chromatic coordinates obtained for the standard solutions of OC (5 mgl⁻¹, 15 mgl⁻¹, 30 mgl⁻¹, 50 mgl⁻¹, and 60 mgl⁻¹), and three replicates of each standard solution were measured. The measurement error was evaluated by $st / \sqrt{n}$, where s is the standard deviation, *t* is the *student-t* at 95% probability and *n-1* freedom degrees, and *n* is the number of measures (3).

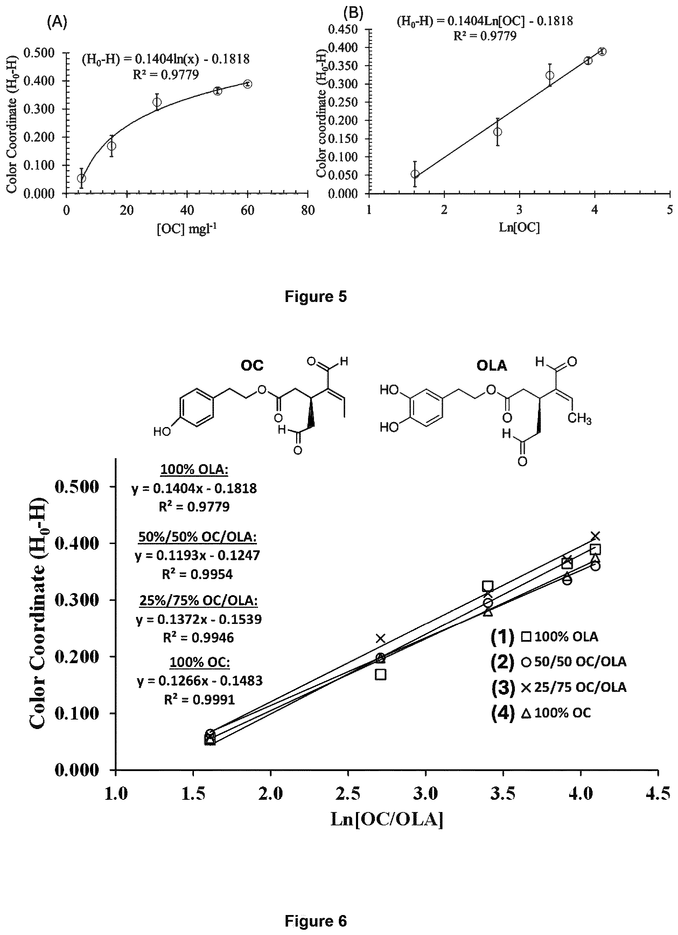

Of all the color coordinates shown in **Fig. 4****,** the H-coordinate was selected as it is the best fit to a simple mathematical model (logarithmic) with a higher R² value than the fit of the rest of the color coordinates. **Fig 5** shows the logarithmic calibration curve as well as its linear representation ((H₀-H) vs Ln [OC]). The detection (LDO) and quantification (LOQ) limits were 29.87 mg kg-1 and 51.26 mg kg-1, respectively. LDO and LOQ were determined using the IUPAC method (LOD=3 s_{b}/m; LOQ=10 s_{b}/m). The standard deviation of the blank (s_{b}) was obtained by measuring (10 measurements) the optical response of Op-Sens-Mat-PEI to the control (0 mgl⁻¹ of OC).

*5.4. Study of the optical response of* Op-Sens-Mat-PEI *to the concentration* of *OLA and OC*/*OLA mixtures.* In this section, calibration curves of Op-Sens-Mat-PEI against the concentration of OLA, and OC/OLA mixtures were performed following the same protocols described in sections 5.1, 5.2, and 5.3. Following calibration curves were prepared following the protocol described in section 5.2:
- *Calibration curve 1*. This calibration curve is performed using 6 standard solutions of 100% OLA: 0 mgl⁻¹ (control), 5 mgl⁻¹, 15 mgl⁻¹, 30 mgl⁻¹, 50 mgl⁻¹, and 60 mgl⁻¹.
- *Calibration curve 2.* This calibration curve is performed using 6 standard solutions based on mixtures OC/OLA 50/50 w/w with the following final concentrations: 0 mgl⁻¹ (control), 5 mgl⁻¹, 15 mgl⁻¹, 30 mgl⁻¹, 50 mgl⁻¹, and 60 mgl⁻¹.
- *Calibration curve 3.* This calibration curve is performed using 6 standard solutions based on mixtures OC/OLA 75/25 w/w with the following final concentrations: 0 mgl^{- 1}(control), 5 mgl⁻¹, 15 mgl⁻¹, 30 mgl⁻¹, 50 mgl⁻¹, and 60 mgl⁻¹.
- *Calibration curve 4.* This calibration curve is performed using 6 standard solutions of 100% OC: 0 mgl⁻¹ (control), 5 mgl⁻¹, 15 mgl⁻¹, 30 mgl⁻¹, 50 mgl⁻¹, and 60 mgl⁻¹.

**Fig.6****.** Shows the calibration curves 1, 2, 3, and 4.

Both the slope and the ordinate at the origin of calibration curves 1, 2, 3, and 4 reported in **Fig. 6** are practically similar, indicating that the optical response (change in the color coordinate H) of Op-Sens-Mat-PEI to OC and OLA is similar. It is probably because OC and OLA are chemically very similar: the only difference between OC and OLA is the hydroxyl group on the aromatic ring (see **Fig. 5**). This difference in the hydroxyl group of the aromatic ring does not influence the imines (Schiff bases) formation through the reaction between the aldehyde groups of OC and OLA and the amino groups of Op-Sens-Mat-PEI. Therefore, all the calibration curves of **Fig. 5** can be used to quantify the sum of the concentrations of OC and OLA ([OC +OLA]) in olive oil samples.

### 6. Real-world application and validation

To evaluate the efficiency in the analysis of real samples, the sum of the concentrations of OC and OLA ([OC +OLA]) of 26 samples of extra virgin olive oil of different origins were analysed by the method proposed in this invention, and the results were compared with those obtained by an accredited reference laboratory (Tello laboratory Jaen: https://www.laboratoriotello.com/ from Tentamus company: https://www.tentamus.es/) using the official reference method(High-performance liquid chromatography: HPLC).

The calibration curve used to measure the concentration of OC and OLA ([OC+OLA]) in the olive oil samples with the method of this invention was established using all the data from calibration curves 1, 2, 3, and 4 in **Fig. 6****.**

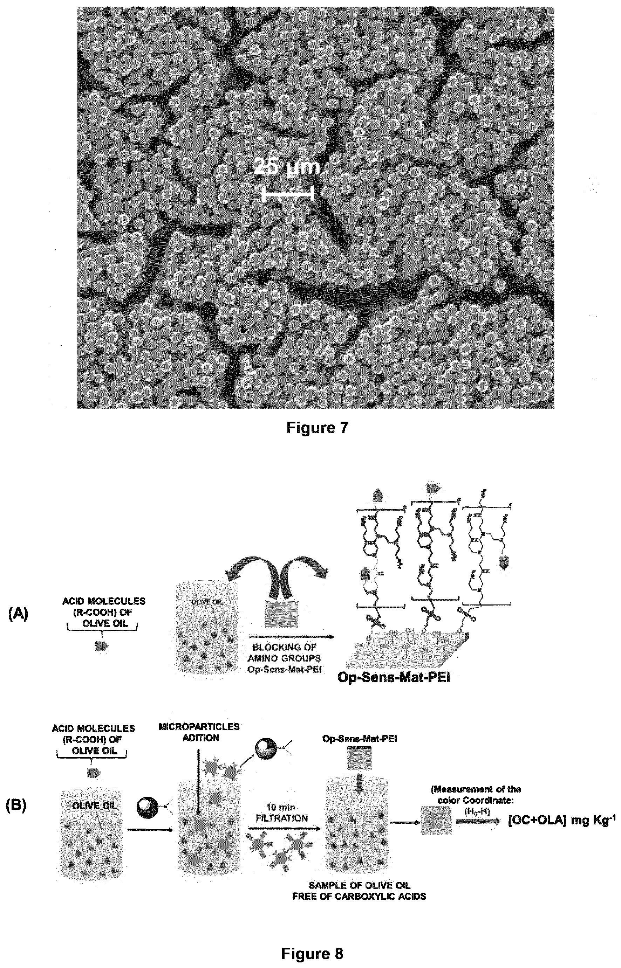

Due to the basic nature of Op-Sens-Mat-PEI, some of its amino groups are blocked by the natural carboxylic compounds present in olive oil samples through acid-base interactions, which introduces an error into the analysis result. To avoid this problem, it is necessary to pre-treat the olive oil samples to remove the acid compounds (R-COOH) that block the amine groups of Op-Sens-Mat-PEI. For this purpose, strongly basic polymeric microparticles functionalized with tertiary amine groups (Part-DVB-N-(CH₃)₂) were synthesised according to the following precipitation polymerization **[6, 7]** protocol: First, the monomer divinylbenzene (DVB) is purified using a chromatographic mini-column (10 ml) of basic alumina. Then, 1.73 ml of DVB, 1.33 ml of 2-(Dimethylamino) ethyl methacrylate (DAEM), and 62.5 mg of 2,2'-Azobis(2-methylpropionitrile) (AIBN) are dissolved in a mixture of solvents composed of 18.3 ml of toluene (TOL), 76.3 ml of acetonitrile (ACN), and 5.3 ml of 2-propanol (2-PrOH) and the mixture is homogenized using a sonicator/vortexer until the reagents are completely dissolved. Subsequently, the solution is purged with N₂ for 5 min to remove dissolved O₂ and the polymerization reaction is carried out under shaking at 62°C for 24 h. Finally, the particles (Part-DVB-N-(CH₃)₂) are centrifuged and washed two times with 50 ml of methanol (Yield: 1 g of spherical Poly-DVB-N(CH₃)₂ of 5 microns in diameter (see **Fig.7**).

*Pretreatment of olive oil samples with Part-DVB-N-(CH₃)₂.* To eliminate the carboxylic compounds present in olive oil samples that block the amino groups of Op-Sens-Mat-PEI, the following protocol is followed: 30 mg of Part-DVB-N-(CH₃)₂ are added to a mixture consisting of 0.25 ml olive oil sample and 1.5 ml THF, then the mixture is stirred at room temperature for 10 min and finally filtered through a 0.5 micron syringe filter to remove particles. **Fig.8** shows a schematic of the olive oil sample treatment protocol.

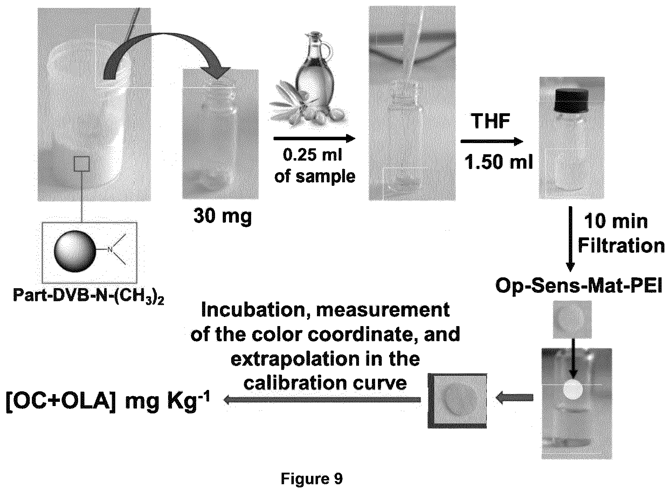

After pre-treatment of the olive oil samples with Part-DVB-N-(CH₃)₂ to remove carboxylic compounds, the quantification of OC and OLA concentration is performed exactly as described in section 5 for the establishment of the calibration curve: 17 µl of acetic acid are added to the mixture composed of 0.25 ml olive oil (free of carboxylic compounds) and 1.5 ml THF and the solution is homogenized for a few seconds by vortexing. Subsequently, a circular piece of 7 mm diameter of Op-Sens-Mat-PEI is introduced in the sample and incubated under shaking for 50 min at 60°C. After incubation, the membrane is washed with 1 ml of hexane for 20 s to remove oil residues, and the color coordinate H is measured as specified in **section 5.3** (see **Fig.3**). Finally, the value of the sum of the concentration of OC and OLA ([OC+OLA]) expressed in mg of (OC +OLA) per kg of olive oil is obtained by extrapolation of the colorimetric measurement (H₀-H) of the membrane incubated in the sample on the calibration curve, and subsequent application of the dilution factor (the operation was repeated three times for each sample). **Fig. 9** shows a complete schematic of the protocol for quantifying the concentration of OC and OLAC ([OC+OLA]) in the EVOO samples.

The validation of the method proposed in this invention was carried out by analysing the total concentration of OC and OLA ([OC + OLA]) of 26 samples of extra virgin olive oil (EVOO) from different origins and comparing the results with those obtained by an accredited reference laboratory (Laboratorio Tello Jaén: https://www.laboratoriotello.com) using the official reference method (HPLC). The results of the validation are shown in **Table 2.**

**Table.2. Comparison of the analysis of OC and OLA concentration in EVOO samples by the method proposed in this invention and the official reference method (HPLC).**

| **Ref.** | **[OLE + OLC] (mg Kg⁻¹) by HPLC reference method** | **[OLE + OLC] (mg Kg⁻¹) by the Method of the invention** |
|---|---|---|
| **15451** | 181.90 ± 18.20 | 188.50 ± 17.30 |
| **15488** | 61.10 ± 6.11 | 79.90 ± 10.60 |
| **15489** | 46.00 ± 4.60 | 59.20 ± 7.50 |
| **15490** | 86.80 ± 8.68 | 103.46 ± 13.90 |
| **15491** | 100.40 ± 10.04 | 103.73 ± 13.80 |
| **15492** | 58.50 ± 5.85 | 69.90 ± 4.00 |
| **15626** | 176.80 ± 17.68 | 158.30 ± 13.80 |
| **15674** | 246.30 ± 24.63 | 248.90 ± 18.20 |
| **15688** | 150.20 ± 15.02 | 137.31 ± 4.70 |
| **15689** | 201.70 ± 20.17 | 192.17 ± 5.14 |
| **15690** | 216.10 ± 21.61 | 230.00 ± 17.28 |
| **15691** | 135.80 ± 13.58 | 126.70 ± 7.40 |
| **15837** | 162.50 ± 16.25 | 158.05 ± 17.50 |
| **15838** | 156.70 ± 15.67 | 150.80 ± 15.60 |
| **15936** | 116.30 ± 11.63 | 96.30 ± 6.30 |
| **15941** | 76.00 ± 7.60 | 81.60 ± 4.11 |
| **15961** | 89.90 ±8.99 | 85.50 ± 15.70 |
| **15962** | 122.20 ± 12.22 | 101.70 ± 10.90 |
| **16198** | 11.20 ± 1.12 | 32.00 ± 7.70 |
| **16210** | 167.20 ± 16.72 | 151.30 ± 10.80 |
| **16363** | 47.80 ± 4.78 | 58.73 ± 7.80 |
| **16364** | 65.00 ± 6.50 | 56.00 ± 14.30 |
| **8269** | 95.90 ± 9.59 | 108.10 ± 5.50 |
| **8272** | 89.00 ± 8.90 | 81.10 ± 12.80 |
| **8270** | 105.20 ± 10.52 | 100.60 ± 8.50 |
| **8271** | 73.40 ± 7.34 | 85.30 ± 12.60 |

The results reported in **Table 2** show that the optical technology presented in the invention allows an accurate, direct, simple, and selective quantification of the concentration of OC and OLA in olive oil samples.

## Claims

1. An optical method for the direct and selective measurement of the concentration of OC and OLA in a liquid sample which comprises:
- to contact the liquid sample with a calibrated optically sensitive material
- to measure a change in an optical property of the optically sensitive material during a constant reaction time and temperature
- to obtain from the change measured in the preceding step in the optical property, the total concentration of OC and OLA of the sample using the calibration curve of the optically sensitive material.

2. The method according to claim 1, which comprises to carry out a sample pre-treatment to remove carboxylic compounds present in the liquid sample, before contacting the sample with the calibrated optically sensitive material.

3. The method according to claim 1 or 2, wherein the optically sensitive material comprises reactive functional groups selected among amine, thiol, carboxy, hydroxyl and combinations thereof, preferably, amine groups coming from molecules selected among: ethylenediamine, propylenediamine, poliethylenimine, poly(ethylene glycol) bis(amine), hexamethylenediamine, p-phenylenediamine, m-xylylenediamine, tris(2-aminoethyl)amine, diethylenetriamine, triethylenetetramine, 3,3'-diamino-N-methyldipropylamine, poly(allylamine), spermidine, cadaverine, spermine, thermospermine, caldopentamine, caldohexamine, branched and hyperbranched polyamines, aromatic amines, polycyclic aromatic amines, hydroxyl amines, thiol amines and carboxyl amines.

4. The method according to claim 1, wherein the optically sensitive material and the sample are allowed to react under one or both or the following provisions:
- the reaction time is comprised between 15 and 60 min, preferably between 20 and 60 min,
- the temperature of reaction is comprised between 20 and 80°C, preferably, between 20 and 60°C.

5. The method according to any of claims 1 to 4, wherein the optically sensitive material is selected from:
- organic, inorganic or hybrid organic/inorganic materials that intrinsically possess functional groups capable of reacting with the aldehyde groups of OC and OLA to form chemical groups with optical properties
- organic, inorganic or hybrid organic/inorganic materials that do not naturally have functional groups capable of reacting with the aldehyde groups of OC and OLA to form chemical groups with optical properties, as a solid support, functionalized with reactive functional groups capable of react with the aldehyde groups of OC and OLA to form chemical groups with optical properties.

6. The method according to any of claims 1 to 5, wherein the optically sensitive material comprises a solid support selected among:
- porous or non-porous organic,
- porous or non-porous inorganic and
- porous or non-porous hybrid organic-inorganic materials.

7. The method according to any of claims 1 to 6, wherein the optically sensitive material is a solid selected among:
- zero-dimensional materials,
- one-dimensional materials,
- two-dimensional materials and
- three-dimensional materials.

8. The method according to any of claims 5 to 7, wherein the optically sensitive material is selected from 0D, 1D, 2D or 3D materials made of, or comprising: polysaccharides, nylon, polyimides, methacrylates, acrylates, polyacrylamides, polystyrenes, polymethacrylates, polyacrylates, silicones, polyamides, polyesters, polycarbonates, polyurethanes, polyethylenes, polyacetylenes, polypropylenes, polyacrilonitriles, tetrafluoroethylenes, polytetrafluoroethylenes, polyurethanes, polysulfones, polythiophenes, polyanilines, polypyrroles, polybenzimidazoles, polyketones, polyamines, DNA, proteins, colloids,
comprising reactive groups that react with the aldehyde groups of OC and OLA,

9. The method according to claim 8, wherein the optically sensitive material is selected from: 0D, 1D, 2D o 3D materials made of polymers that intrinsically possess amino groups.

10. The method according to claim 1, wherein the optical property of the optically sensitive material is selected among color, transmittance, absorbance, reflectance, luminescence, infrared and Raman spectroscopies, interferometry, and surface plasmon resonance.

11. The method according to claim 1 wherein the optically sensitive material comprises one or more of the following raw inorganic materials: glass, aluminium oxide, silicon dioxide, zirconium oxide, zeolites, carbon, cordierite, silicon carbide, silicon nitride, mullite, bentonite, zeolite, tin oxide, silicon carbide, iron oxide, zinc oxide, silicon, zirconium, copper, titanium, gold, silver, and iron.

12. The method according to claim 1, wherein the optically sensitive material is a hybrid material selected from:
- Metal Organic Framework (MOF),
- composite materials based on inorganic micro or nanoparticles dispersed in organic polymers or vice versa.

13. The method according to claim 1, wherein the calibration of the optically sensitive material is performed in a liquid matrix free of OC and OLA selected among refined olive oil, sunflower oil, triolein, water, organic solvents: methanol, ethanol, acetonitrile, hexane, mixtures of organic solvents, and mixtures of organic solvents and water.

14. The method according to claim 1, wherein the liquid sample is selected among vegetable oil, aqueous extracts of plants or fruits and organic extracts of plants or fruits.

15. A device for carrying out the method defined in any of claims 1 to 14, for the direct and selective measurement of the concentration of OC and OLA in liquid samples which comprises:
- an optically sensitive material suitably calibrated for the optical measurement of OC and OLA.
- means to bring the optically sensitive material into contact with the liquid samples,
- a thermostatic device with stirring to carry out the reaction between the functional groups of the optically active material and the aldehyde groups of OC and OLA at a controlled temperature between 20°C and 80°C, for a time between 15 and 50 min. an optical measurement device capable of measuring the change in the optical property of the optically sensitive material after interaction with the sample
- means to execute a computer program capable of directly relating the change in the optical property of the optically sensitive material to the concentration of OC and OLA in the sample.
